Europäisches Patentamt

European Patent Office

Office européen des brevets

(11) Publication number: **0 022 640**
A1

# EUROPEAN PATENT APPLICATION

(21) Application number: 80302241.7

(22) Date of filing: 02.07.80

(30) Priority: 09.07.79 AU 9493/79
01.02.80 AU 2199/80

(43) Date of publication of application: 21.01.81
Bulletin 81/3

(84) Designated Contracting States: BE DE FR GB IT NL

(51) Int. Cl.³: **B 01 J 29/28,** C 07 C 11/04,
C 07 C 1/24, C 07 C 1/20,
C 07 C 2/00

(71) Applicant: **Commonwealth Scientific and Industrial
Research Organization, Limestone Avenue, Campbell
Australian Capital Territory, 2601 (AU)**

(72) Inventor: **Anderson, John Robert, 8 Novello Court,
Eltham, Victoria 3095 (AU)**
Inventor: **Rajadhayaksha, Rajeev Anand, Miranda House
Veer Sawarkar Path Thane, Maharashtra, 400602 (IN)**
Inventor: **Weiss, Donald Eric, 3 Cornish Place, Holder,
A.C.T.2611 (AU)**
Inventor: **Mole, Thomas, 2 Maxwell Street, Kew 3101,
Victoria (AU)**
Inventor: **Wilshier, Keith George, 23 Blackwood Drive,
Mulgrave 3170 Victoria (AU)**
Inventor: **Whiteside, Judy Anne, 14 Highclere Street,
Warragul 3820 Victoria (AU)**

(74) Representative: **Collier, Jeremy Austin Grey et al,
J.A.Kemp & Co. 14, South Square Gray's Inn, London
WC1R 5EU (GB)**

(54) **Improved zeolite catalysts, method for their preparation and their use in the conversion of ethanol into ethylene and of both into higher hydrocarbons.**

㊗ An improved zeolite catalyst for use in, and a method of treating a zeolite catalyst to improve its performance in, the conversion of ethanol and/or ethylene to higher hydrocarbons containing from three to twelve carbon atoms comprising treating a zeolite having a $SiO_2$ to $Al_2O_3$ ratio greater than 10 and being a ZSM or related type zeolite with a hydrogen halide, an organic halide capable of elimination of hydrogen halide, or a mineral acid. The treatment of the zeolite to improve its performance may occur simultaneously with, or prior to, the conversion of ethanol and/or ethylene to the higher hydrocarbons at a temperature up to 500°C and a pressure of from 1 to 10 atmospheres for up to 70 hours. The conversion of ethanol to ethylene is also within the scope of the present invention. The industrial applicability of the present invention resides in the production of higher hydrocarbons which may be used as fuel or fuel supplements.

- 1 -

"Improved Zeolite Catalysts, Method for their preparation, and their use in the Conversion of Ethanol into Ethylene and of both into higher hydrocarbons".

This invention relates to improved zeolite catalysts, to methods of producing such catalysts, and to their use in the conversion of ethanol and ethylene to liquid and aromatic hydrocarbons, including the conversion of ethanol to ethylene. More particularly this invention relates to the use of zeolite catalysts of high $SiO_2:Al_2O_3$ ratios such as the ZSM and related types in the conversion reaction of aqueous and anhydrous ethanol to ethylene, of aqueous ethanol to higher hydrocarbons, and of ethylene into liquid and aromatic hydrocarbons.

Zeolites of the ZSM type, in particular ZSM-5, are known to be of value as catalysts for the conversion of a range of gaseous and liquid organic materials such as olefins or oxygen containing compounds into hydrocarbons, including both liquid and aromatic hydrocarbons. See for example the following references.

1.   C. D. Chang and A. D. Silvestri, J. Catalysis 47 (1977) 249 et seq.
2.   J. R. Anderson, K. Foger, T. Mole, R. A. Rajadhyaksha and J. V. Sanders, J. Catalysis 58 (1979) 114 et seq.

BAD ORIGINAL

3.     Mobil Oil Corp, Australian Patents 465,697; 479,875; 487,963; 488,590 and references.

4.     R. A. Rajadhyaksha and J.R. Anderson, J. Catalysis (accepted for publication).

5.     Australian Patent Application PD9493/79.

One attraction of the conversion of gaseous and liquid organic materials into hydrocarbons lies in the fact that under suitable conditions much of the hydrocarbon product is of a boiling range suited to a motor spirit and much consists of branched chain compounds and simple benzenoid compounds.

The value of the catalysts used in such conversion reactions derives firstly from the selectivity with which hydrocarbons containing 5 or more carbon atoms are formed, and more so from the selectivity with which benzenoid hydrocarbons of the benzene, toluene, xylene and tri-methylbenzene series are formed. In both these respects the catalysts are suitable for the manufacture of gasoline and aromatics from starting materials which include the lower olefins, alcohols and other oxygenated compounds. Typically, high conversions of such compounds are obtained by passing the vapour of the compounds with or without diluent gas over catalysts at a temperature generally in the range 350 to 400$^{o}$C.

Methanol, for example, can be converted to hydrocarbons quite readily by passing it as a vapour with or without diluent gas over a ZSM catalyst at a temperature of about 340 to 400$^{o}$C. As methanol can be produced from synthesis gas by well established and relatively simple technology, and as synthesis gas is readily obtained by steam reforming of methane or by the action of steam and oxygen on coal or coke, the combination of these processes with catalytic conversion of the methanol provides an attractive route for production of high octane motor spirit. However, methanol is not a particularly desirable fuel for direct use as motor spirit because it has relatively low calorfic value and poor miscibility with petrol at low temperatures and in moist conditions.

Of the simple alcohols, methanol as described above and other alcohols which contain more than two carbon atoms readily undergo the conversion to the higher hydrocarbons, but not so with ethanol as is explained below. Propylene and all the butenes undergo ready conversion of the kind described above. However, of the simple olefins, only ethylene does not reliably undergo such high conversion. In contrast to methanol, the direct use of ethanol as a motor spirit is not greatly limited by the same factors which restrict the use of methanol as has previously been described. However, the use of ethanol as motor spirit has hithertobefore been limited by other factors such as restrictions and taxation on its production, sale and consumption. These imposed limitations can be overcome by the conversion of ethanol to ethylene and to higher hydrocarbons suitable for use in motor spirit.

As already mentioned, anhydrous ethanol, unlike methanol, has not been found to undergo reliable conversion to higher hydrocarbons such as liquid and aromatic hydrocarbons. Depending on conditions, conversion of ethanol over samples of the proton form of the ZSM-5 zeolite obtained by way of the ammonium form over a period of 2 to 4 hours gives as the main products:-

(a) ethylene with no other hydrocarbons, or

(b) ethylene and aromatic compounds containing 10 or more carbon atoms, or

(c) $C_6$ to $C_{10}$ aromatic hydrocarbons.

The desirable behaviour (c) was uncommon and could not be reliably reproduced after regenerating the catalyst in oxygen or hydrogen or in both. However, reaction (c) can be promoted by first activating the ZSM-5 catalyst by treatment with hydrogen halides, organic halides or aqueous mineral acids. Those skilled in the art would appreciate that other zeolites of high $SiO_2:Al_2O_3$ ratio might be likewise activated.

Ethylene, which may be produced by the dehydrogenation of ethanol, also does not undergo ready conversion to $C_6$ to $C_{10}$ aromatics over the proton form of ZSM-5 zeolite obtained by way of the ammonium form but the conversion can be promoted by the use of zeolite catalyst activated in the same manner as described above in relation to the conversion of ethanol.

Therefore, it is one object of the present invention to overcome at least some of the problems inherent in the prior art by providing an improved zeolite catalyst.

A further object is to provide a method of making the improved zeolite catalyst.

A still further object is to provide methods of use of the improved catalyst whereby anhydrous or aqueous ethanol may be converted to ethylene and whereby aqueous ethanol and ethylene may be converted to hydrocarbons.

In accordance with one aspect of the present invention there is provided a zeolite catalyst for use in the conversion of ethanol to ethylene and higher hydrocarbons and of ethylene to higher hydrocarbons, characterized in that it comprises a zeolite having a $SiO_2$ to $Al_2O_3$ ratio greater than 10 and being of the ZSM or a related type, which has been treated with a hydrogen halide, an organic halide capable of elimination of hydrogen halide or a mineral acid to improve the catalytic properties of the zeolite.

A second aspect of the present invention provides a method of preparing a zeolite catalyst for use in the conversion of ethanol to ethylene and higher hydrocarbons and of ethylene to higher hydrocarbons, characterised in that a zeolite having a $SiO_2$ to $Al_2O_3$ ratio greater than 10 and being of the ZSM or related type is treated with a hydrogen halide, an organic halide capable of elimination of hydrogen halide or a mineral acid to improve the catalytic properties of the zeolite.

A third aspect of the present invention provides a method of converting ethanol and ethylene to higher hydrocarbons and/or of converting ethanol to ethylene wherein the ethanol or ethylene is contacted with a zeolite catalyst, characterized in that the catalyst comprises a zeolite having a $SiO_2$ to $Al_2O_3$ ratio greater than 10 and being of the ZSM or related type and which prior to or simultaneously with contact with the ethanol or ethylene, is treated with a hydrogen halide, an organic halide capable of elimination of hydrogen halide or a mineral acid to improve the catalytic performance of the zeolite.

Zeolites used in accordance with the present invention are those with $SiO_2$ to $Al_2O_3$ ratios greater than 10 of the ZSM and related types including ZSM-5, ZSM-8, ZSM-11, ZSM-12 and zeolite β.

The zeolites include all those made by the action of tetraalkyl ammonium bases having alkyl groups of 2 or more carbon atoms upon starting material containing the elements of alumina and silica.

When used as catalysts, according to the prior art, such zeolites are converted, most commonly by cation exchange techniques, to forms which display or develop Brönsted acidity under the conditions of catalysts. Most commonly the zeolite, freed of organic matter is exchanged with a solution of an ammonium salt to give the ammonium form of the zeolite which is decomposed to the proton form by heating.

However the method of the present invention applies not only to the ammonium and proton forms of the appropriate zeolites prepared as above, but to these forms however prepared and to forms of the zeolite in combination with multivalent cations which confer Brönsted acidity such as for example the rare earth cations. Furthermore, the method of the invention is not limited to zeolites which are completely exchanged into the cation or Brönsted acidity but also applies to incompletely exchanged forms.

A preferred form of the invention utilizes as the starting material a zeolite which is largely in the hydrogen form but with some residual sodium, which is then activated according to the method of this invention. A particularly preferred form of the invention uses ZSM-5 zeolite activated with 0.1 to 1.0 molar mineral acid at $20^{\circ}$ to $200^{\circ}C$ for 20 to 70 hours.

The second aspect of the present invention, which is the method of treating the zeolite so as to improve its catalytic properties, will now be discussed in more detail.

In one embodiment of the second aspect of the present invention, the zeolite is treated with dilute aqueous mineral acid preferably at a temperature between $0^{\circ}C$ and $100^{\circ}C$ for a period of 1-48 hours prior to use as a catalyst. In this case the mineral acid is preferably hydrochloric acid but may be any other strong mineral acid. In a preferred form of this embodiment, the proton form of ZSM-5 zeolite is treated with 0.5 to 1.0 molar hydrochloric acid at 20 to $40^{\circ}C$ for 20 to 40 hours.

In another embodiment of the second aspect of the present invention, the zeolite is treated with the vapour of a hydrogen halide or of an aqueous hydrogen halide. Such treatment of the zeolite by the hydrogen halide may be effected prior to the use of the zeolite as a catalyst, or may be effected during the use of the zeolite catalyst in the conversion process by introducing the hydrogen halide into the ethylene or ethanol feed, either continuously or intermittently. In one preferred form of this latter embodiment, the untreated proton form of the ZSM-5 type zeolite is employed as a catalyst for the conversion of a 2% w/w solution of HCl in ethanol.

In a further embodiment of the second aspect of the present invention, the zeolite is heated with an organic halide either prior to the use of the zeolite as a catalyst, or during such use of the zeolite.

Suitable organic halides are all those hydrocarbon halides containing from 1 to 20 carbon atoms excluding perhalogenated compounds, such as carbon tetrachloride, which cannot undergo elimination of hydrogen halide. In a preferred form of this embodiment, 1,2-dichloroethane in an amount from 0.1% to 5.0% v/v is added to the ethanol and this solution is then contacted with the proton form of the ZSM-5 type zeolite at a reaction temperature of 300 to 450°C, to convert the ethanol in accordance with the third aspect of the present invention.

As the hydrogen halide and the organic halides may be generated and applied to the treatment of the zeolites according to the method of the invention in diverse ways, it is not intended that the invention be restricted only to those forms of halide generation described herein. Any method of producing halogen-containing compounds or other compounds suitable for treating zeolites of the type described herein may be used.

Also encompassed by this invention is the incorporation of the zeolite into any suitable matriz or support medium which may then be used as a catalyst.

It will be obvious to those skilled in the art that the zeolite may be combined with, dispersed in, or otherwise admixed with a porous matrix or other suitable support media. It should be understood that the method of this invention, whereby the zeolite is treated with a halogen containing compound or mineral acid may be applied to either zeolite itself or to any such zeolite-containing admixture.

An important advantage obtained when using catalysts activated in accordance with the present invention is the lifetime of such catalysts may be readily extended.

The third aspect of the present invention, that is the conversion of ethanol and ethylene into higher hydrcarbons such as liquid and aromatic hydrocarbons and the conversion of ethanol into ethylene, will now be described in more detail.

The higher hydrocarbons which are produced by the conversion of ethanol and ethylene comprise hydrocarbons containing more than 2 carbon atoms and are most usually hydrocarbons containing from 3 to 12 carbon atoms. Such hydrocarbons include saturated and unsaturated hydrocarbons usually containing from 3 to 6 carbon atoms, benzene, toluene, xylene, tri-methylbenzenes and other aromatic hydrcarbons containing 12 or less carbon atoms, all of which are useful components for motor spirit and chemical feedstuffs.

Whether the product of the conversion of ethanol will be predominantly ethylene or higher hydrocarbons or a mixture thereof depends on a number of reaction variables which include catalytic activity, temperature, water content of the ethanol and ethanol feed rate. For example, a decrease in the treatment temperature promotes the formation of ethylene while an increase in the temperature promotes the formation of higher hydrocarbons. For a given reaction temperature, the more "active" the catalyst, the more likely it is that the products will be higher hydrocarbons rather than ethylene. The activity of the catalyst depends on such factors as composition, method of preparation and activation. The importance of such factors is well known to those skilled in the art and the conditions are selected with regard to the products desired taking into account such known factors.

In one embodiment of the third aspect of the present invention hydrocarbons with carbon numbers greater than two including liquid and aromatic hydrocarbons are produced by conversion of aqueous ethanol (4-96 volume % ethanol) with the activated zeolite at relatively high temperatures (about $250^{\circ}$ to $500^{\circ}$C). In a preferred form of this embodiment the activated form of ZSM-5 is treated with 0.2 to 50 parts by weight of aqueous ethanol per part by weight of catalyst per hour at a pressure of one to 10 atmospheres.

In another embodiment of the third aspect of the present invention ethylene is produced by conversion of aqueous ethanol (4-96 volume % ethanol) with the activated zeolite at relatively low temperatures (about $150^{\circ}$ to $300^{\circ}$C). In a preferred form of this embodiment the activated form of ZSM-5 is treated with 0.2-50 parts by weight of aqueous ethanol per part by weight of catalyst per hour at a pressure of 1 to 10 atmospheres.

In a further embodiment of the third aspect of the present invention ethylene is produced by conversion of anhydrous ethanol with the activated zeolite at relatively low temperatures (about $150^{\circ}$ to $300^{\circ}$C). In a preferred form of this embodiment the activated form of ZSM-5 is treated with 0.2 to 50 parts by weight of anhydrous ethanol per part by weight of catalyst per hour at a pressure of 1 to 10 atmospheres.

In the first two embodiments of the third aspect of the present invention previously described, the water content of the ethanol may cover a wide range (4 to 96 volume % water). For economic reasons a range with lower water contents may be preferred.

Aqueous or anhydrous ethanol can be brought into contact with the activated zeolite in diverse ways, for instance, the ethanol in vapour form may be brought into contact alone or may be carried by an inert gas or recycled gas into contact with the catalyst which can be used as a fixed or fluidised bed. It is not intended that the invention be restricted only to the forms of treatment described herein.

The performance of zeolite catalysts change with time during the catalytic conversion. Thus, the composition and distribution of conversion products can vary with time. The initial activity can, however, be restored by regeneration of the catalyst. One such regeneration treatment involves exposing ZSM-5 in a stream of oxygen for 16 hours at 500°C (ref. 4.). In the present case, it has been found that the catalysts can be regenerated by a similar high temperature treatment in oxygen. Those skilled in the art will be aware that other regeneration procedures may be applied, for instance, exposure to a stream of air at high temperature. Advantage may also attach to an additional periodic reactivation if the feed material for catalytic conversion is aqueous or anhydrous ethanol originating from a fermentation process containing dissolved mineral matter.

Those skilled in the art will appreciate that in processes such as those described, products such as $C_3$ hydrocarbons (e.g. propane) and $C_{4-5}$ hydrocarbons (e.g. isobutane and n-butane) can be converted to aromatic hydrocarbons by known conversion processes using catalysts such as ZSM-5. Thus, the present invention may be used in combination with such known processes to produce hydrocarbons having the required number of carbon atoms.

The various aspects of the present invention will now be further described with reference to the following examples which, however, should not be taken as limiting the invention in any way.

EXAMPLE 1 - <u>Illustrating the Preparation and Activation</u>
<u>of Zeolite Catalyst and its Use in the</u>
<u>Conversion of Ethanol</u>

Zeolite catalyst was prepared by the following method:
90 parts by weight of sodium silicate solution having the composition 27.9 wt.% $SiO_2$, 8.7 wt.% $Na_2O$ and 63.4 wt.% $H_2O$ was mixed with 110 parts of water and 10 parts of n-propanol. A second solution containing 3 parts of aluminium sulphate hydrate ($Al_2(SO_4)_3.18H_2O$), seven and one half parts of 98 wt.% sulphuric acid and 150 parts of water was rapidly mixed with the first solution. The mixture was shaken vigorously for 5 minutes to produce a firm gel which was heated in a clean, closed stainless steel pressure vessel at 175°C for 24 hours. The heating was interrupted after the first 2 hours in order to shake the mixture vigorously for 2 minutes. After 24 hours, solid product was removed from the pressure vessel, filtered, washed copiously with distilled water and dried at 120°C for 4 hours. The zeolite then had the mole ratio composition of $Na_2O:Al_2O_3:SiO_2 = 1:1:48$.

The white crystalline product was activated as follows:
It was first calcined in air at 500°C, then refluxing gently with 150 parts of 0.3 molar hydrochloric acid for 24 hours. It was finally filtered, washed copiously with distilled water, and then dried in air at 120°C. The X-ray diffraction pattern of the final catalyst was similar to that reported for orthorhombic ZSM-5 by E L Wu etal, in 83 <u>J Phys.Chem</u> (1979) 2777 and did not indicate the presence of any other compounds. The zeolite so activated had a mole ratio composition of $Na_2O:Al_2O_3:SiO_2 = 0.03:1:48$.

0.15g of the catalyst was packed into a quartz microreactor tube, pre-treated with oxygen at 500°C, and fed with dry ethanol at a rate of 0.8g/hour and nitrogen at a rate of 2.2ml/minute at a reactor
temperature of 280°C under a pressure of 1-2
atmospheres. Complete conversion of the ethanol to
hydrocarbons and water occurred. Initially the organic
product contained 45% of aromatic hydrocarbons of carbon
number 6-9 (particularly toluene and xylenes) and 2%
ethylene. Most of the balance of the organic products
consisted of aliphatic hydrocarbons of carbon numbers
3-6.

During two hours, conversion was complete but the
percentage of $C_{6-9}$ aromatic hydrocarbons fell 25% and that
of ethylene rose to 16%. The catalyst could be restored
to its original appearance and activity by regeneration in
a stream of oxygen at 500°C for 1 hour.

EXAMPLE 2 -  The Use of a Zeolite Catalyst for the
             Conversion of Ethanol to Higher Hydrocarbons

An experiment was carried out like that of example 1
but at a reactor temperature of 420°C. The ethanol was
completely converted to hydrocarbons and water. Initially
50% of the hydrocarbons consisted of $C_{6-9}$ aromatic
hydrocarbons and 2% of ethylene, with aliphatic hydrocarbons of carbon number 3-6 making up most of the
balance. After five hours on stream the proportion of
atomatic hydrocarbons of carbon number 6 to 9 in the hydrocarbon
product was about 30% and the ethylene about 7%. The
appearance and activity of the catalyst could be restored by
regeneration in oxygen at 500°C for 1 hour.

EXAMPLE 3 -  The Use of a Zeolite Catalyst for the
             Conversion of Aqueous Ethanol to Mainly
             Higher Hydrocarbons

An experiment was carried out just like that of Example
2 except in that the feed consisted of 1.0g/hour of
aqueous ethanol (3vol $H_2O$:1volEtOH). Conversion of the ethanol to hydrocarbons consisted of $C_{6-9}$ aromatic
hydrocarbons and 9% of ethylene. After 1½ hours 28%
of $C_{6-9}$ aromatic hydrocarbons and 13% of ethylene,
the balance of the hydrocarbons consisted mainly of $C_{6-9}$
aliphatic hydrocarbons. The catalyst could be
restored to its initial appearance and activity by
regeneration in a stream of oxygen at 500°C for 1 hour.

EXAMPLE 4  -  A Further Preparation of a Zeolite
Catalyst, its activation and Use in the
Conversion of Ethanol to Ethylene

Zeolite catalyst was prepared by the following method:
A mixed solution of 90 parts by weight of sodium silicate
having the composition 27.9 wt% $SiO_2$, 8.7 wt% $Na_2O$ and
63.4% $H_2O$ was mixed with 110 parts of water and 10
parts of n-propanol. A second solution containing 9
parts of aluminium sulphate hydrate, 4.5 parts of 98%
sulphuric acid and 150 parts of water was rapidly
mixed with the first solution. The mixture was shaken
vigorously for 5 minutes to give a gel which was heated
in a clean, closed stainless steel pressure vessel for
48 hours at 177°C. The heating was interrupted after
the first two hours to shake the vessel vigorously for two
minutes. After 48 hours the product was filtered, washed
for two minutes. After 48 hours the product was
filtered, washed copiously with distilled water, dried
at 120°C for 4 hours and calcined at 500°C. It then
had the mole ratio composition $Na_2O:Al_2O_3:SiO_2$ of 1:1:22
and had a diffraction pattern similar to that of the
material produced by the method of example 1. 1 part of
the calcined zeolite was refluxed with 150 parts of
0.3 molar hydrochloric acid for 24 hours, then filtered,
washed copiously with water and dried at 120°C. The
product was of unchanged crystallinity and had a mole
ratio composition $Na_2O:Al_2O_3:SiO_2$ of 0.01:1:22.

The catalyst was pelleted. 0.15g of broken pellets of
80-100 mesh size, packed into a microreactor tube was first heated to 500°C in air and then fed with
anhydrous ethanol at a rate of 0.8g/hour and nitrogen
at a rate of 2.2 ml/minute.  At a microreactor
temperature of 248°C and pressure of 1-2 atmospheres,
complete dehydration of ethanol to ethylene occurred
during a period of 3 hours.

EXAMPLE 5   -   The Use of a Zeolite Catalyst for the
                Conversion of Ethanol to Higher Hydrocarbons

An experiment was carried out just like that of
Example 4 except in that the microreactor temperature
was 291°C.  Complete conversion of ethanol to water
and hydrocarbons occurred.  Less than 2% of the product
was ethylene, 40% was hydrocarbons of carbon numbers
3-4 and 44% was aromatic hydrocarbons of carbon numbers
6-9 (mainly toluene and xylenes).

EXAMPLE 6   -   A Further Preparation of a Zeolite Catalyst,
                its Activation and Use in the Conversion
                of Ethanol to Ethylene

Zeolite catalyst was prepared by the following method:
90 parts by weight of sodium silicate solution having
the composition 27.9 wt.% $SiO_2$, 8.7%wt.% $Na_2O$ and
63.4 wt.% water was mixed with 80 parts water, 10 parts
of  n-propanol and 40 parts of 25 wt.% ammonia solution.
A second solution containing 3 parts of aluminium
sulphate hydrate $(Al_2(SO_4)_3.18H_2O)$, 7.5 parts of 98 wt. %
sulphuric acid and 150 parts of water was rapidly mixed
with the first solution.  The resulting mixture was
heated in a clean, closed stainless steel pressure
vessel according to the method of example 1.  The
product was washed and dried in accordance with example 1
and had a diffraction pattern similar to that of the
material produced by the method of example 1.  The
product was calcined and treated with aqueous hydrochloric acid according to the method of example 1.

0.15g of the catalyst so produced and activated was packed into a quartz microreactor. Dry ethanol was fed to the reactor at a rate of 0.8g/hour and nitrogen at a rate of 2.2ml/minute at a reactor temperature of 290$^{\circ}$C and a pressure of 1-2 atmospheres. 100% dehydration of the ethanol to ethylene occurred and was maintained for a period of 1 hour. The catalyst discoloured slightly but was restored to its original condition and activity by regeneration in a stream of oxygen at 500$^{\circ}$C for 1 hour.

EXAMPLE 7 - The Use of a Zeolite Catalyst for the
Conversion of Aqueous Ethanol to Ethylene

0.15 g of activated catalyst as described in example 4 was treated with a feed consisting of 0.9g/hour of aqueous ethanol (1:1 v/v) in nitrogen carrier gas at a rate of 2.2ml/minute according to the method of Example 6 but at a reactor temperature of 240$^{\circ}$C. The ethanol was completely converted to hydrocarbons and water and the hydrocarbon product consisted of 98% ethylene plus 2% higher hydrocarbons.

EXAMPLE 8 - A Further Illustration of the Conversion
of Aqueous Ethanol

The experiment of Example 7 was repeated at a reactor temperature of 290$^{\circ}$C. The ethanol was completely converted to hydrocarbons and water and the hydrocarbon product consisted of 50-605 ethylene and 40-50% higher hydrocarbons.

EXAMPLE 9

Ethanol containing 4w/w 1,2-dichloroethane was fed at a rate of 0.7g/hr via a preheater coil to a microreactor containing 0.1g of the proton form ZSM-5 catalyst, prepared and activated as described in Example 1 at a temperature of 400°C and a total pressure of 1 atmosphere.
During the first three hours of reaction, ethanol
conversion was greater than 95%, and more than 80%w/w
of the organic product consisted of aromatic products
in the $C_6$-$C_{10}$ molecular size range, with less than
20% in the greater than $C_{10}$ size range.  After 5
hours ethanol conversion was still more than 95%,
but approximately 50%w/w of the product was in the
greater than $C_{10}$ size range.

At this stage regeneration of the catalyst in an
oxygen stream at 400°C for not more than 16 hour
restored the desirable behaviour observed initially.
Such catalyst use and regeneration could be repeated
at least four times without significant adverse
effect upon the catalytic behaviour.

EXAMPLE 10

The experiment of example 9 was repeated but with an
ethanol feed containing 2%w/w hydrogen chloride.
The desirable behavious observed during the first 3
hours in example 9 was observed during a period of
4 hours at a reactor temperature of 400°C.

EXAMPLE 11

0.13 g of the proton form of ZSM-5 catalyst as described in example 9 was treated with 5ml of 0.6 molar aqueous nitric acid for 25 hours at 25$^{\circ}$C. The catalyst was filtered but not washed, and dried at 110$^{\circ}$C. Upon treatment with pure ethanol feed in the manner described in Example 9, more than 95% conversion of ethanol was observed during the first 2 hours at 400$^{\circ}$C, and more than 80% w/w of the product consisted of aromatic hydrocarbons of $C_6$-$C_{10}$ molecular size.

EXAMPLE 12

0.13g of the proton form of ZSM-5 catalyst was treated with 0.6 molar nitric acid according to the manner described in example 3. This sample of catalyst when tested according to the procedure of example 11 gave results similar to those obtained in Example 11.

EXAMPLE 13

ZSM-5 catalyst, which had been treated with ethanol containing 4% w/w 1,2-dichloroethane according to the method of Example 9, was fed with a mixture of ethylene (30%) and hydrogen (70%) at a gaseous feed rate of 8.5cc/min. The conversion of ethylene was 80-90% at a reactor temperature of 400$^{\circ}$C. 50%w/w of the product consisted of aromatic hydrocarbons of $C_6$ to $C_{10}$ molecular size, 2% consisted of aromatic hydrocarbons of greater molecular size, and most of the balance consisted of $C_3$ and $C_4$ hydrocarbons. During the period of 2 hours the selectivity for formation of $C_6$-$C_{10}$ hydrocarbons increased to 80%, and this selectivity was then maintained for at least 4 hours.

Similar results were obtained when the ethylene feed
was admixed with nitrogen.

EXAMPLE 14

ZSM-5 catalyst was treated with ethanol containing
hydrogen chloride according to the method of Example 10.
Ethylene was then fed to the catalyst at 400°C according
to the method of Example 13. The conversion and
selectivity were similar to those observed in Example
12.

EXAMPLE 15

ZSM-5 catalyst activated using aqueous nitric acid
according to the method of Example 11 was treated with
an ethylene containing feed according to the method of
Example 13. The conversion and selectivity were
similar to those observed in Example 13.

EXAMPLE 16

ZSM-5 catalyst activated using aqueous hydrochloric
acid according to the method of example 12 was treated
with an ethylene containing feed according to the
method of Example 13. The conversion and selectivity
were similar to those observed in Example 13.

CLAIMS:

1.      A zeolite catalyst for use in the conversion of ethanol to ethylene and higher hydrocarbons and of ethylene to higher hydrocarbons, characterized in that it comprises a zeolite having a $SiO_2$ to $Al_2O_3$ ratio greater than 10 and being of the ZSM or a related type which has been treated with a hydrogen halide, an organic halide capable of elimination of hydrogen halide or a mineral acid to improve the catalytic properties of the zeolite.

2.      A catalyst as claimed in claim 1, characterized in that the zeolite is selected from the group consisting of ZSM-5, ZSM-8, ZSM-11, ZSM-12 or zeolite β types.

3.      A catalyst as claimed in claim 1 or claim 2, characterized in that the zeolite is partially or wholly in a proton form, an ammonium form, a rare-earth, cation-exchanged form or any combination thereof.

4.      A catalyst as claimed in any one of the preceding claims, characterized in that the zeolite is a ZSM-5 type largely in the hydrogen form with some residual sodium and has been treated with 0.1 to 1.0 molar mineral acid as $20^O$ to $200^O C$ for 20 to 70 hours.

5.      A catalyst as claimed in any one of the preceding claims, characterized in that it is incorporated into any suitable matrix or support medium.

6.      A catalyst as claimed in any one of the preceding claims, characterized in that it has undergone regeneration as well as treatment.

7.      A method of preparing a zeolite catalyst
for use in the conversion of ethanol to ethylene and
higher hydrocarbons and of ethylene to higher hydrocarbons,
characterized in that the zeolite having a $SiO_2$ to
$Al_2O_3$ ratio greater than 10 and being of the ZSM or
related type is treated with a hydrogen halide, an
organic halide capable of elimination of hydrogen
halide or a mineral acid to improve the catalytic
properties of the zeolite.

8.      A method as claimed in claim 7, characterized
in that the zeolite is treated with a hydrogen halide
vapour or an aqueous solution of a hydrogen halide.

9.      A method as claimed in claim 8, characterized
in that the treatment of the zeolite by  the hydrogen
halide is effected prior to use of the zeolite catalyst
in the conversion of ethanol to ethylene and
higher hydrocabons and of ethylene to higher hydrocarbons.

10.      A method as claimed in claim 8, characterized
in that the treatment of the zeolite by the hydrogen
halide occurs during the use of the zeolite catalyst
in the conversion process by introducing the hydrogen
halide into the ethylene or ethanol feed either
continuously or intermittently.

11.      A method as claimed in claim 7, characterized
in that the organic halide is a hydrocarbon halide
of a carbon compound containing from 1 to 20 carbon
atoms, excluding perhalogenated compounds.

12.      A method as claimed in claim 11, characterized
in that the organic halide is 1,2-dichloroethane.

13.      A method as claimed in claim 12, characterized
in that a solution of 1,2-dichloroethane in ethanol
in an amount from 0.1% to 5.0% v/v is contacted with
a proton form of ZSM-5 type zeolite at a temperature
of 300 to 450$^{o}$C.

14.      A method as claimed in claim 7, characterized
in that the mineral acid is hydrochloric acid or
nitric acid.

15.      A method as claimed in claim 7, characterized
in that the zeolite is treated with 0.3 molar
hydrochloric acid for up to 48 hours.

16.      A method as claimed in claim 7, characterized
in that a ZSM-5 type zeolite is treated with 0.5 to
1.0 molar hydrochloric acid at 20 to 40$^{o}$C for 20
to 40 hours.

17.      A method of converting ethanol and ethylene
to higher hydrocarbons and/or of converting the ethanol
to ethylene wherein the ethanol or ethylene is contacted
with a zeolite catalyst, characterized in that the
catalyst comprises  a zeolite having a $SiO_2$ to $Al_2O_3$
ratio greater than 10 and being of the ZSM or related
type and which prior to or simultaneously with contact
with the ethanol or ethylene is treated with a hydrogen
halide, an organic halide capable or elimination of
hydrogan halide or a mineral acid to improve the
catalytic performance of the zeolite.

18.      A method as claimed in claim 17, characterized
in that the catalyst is as claimed in any one of claims
2 to 5 .

19.    A method as claimed in claim 17 or claim
18, characterized in that aqueous ethanol is converted
to ethylene or higher hydrocarbons or both.

20.    A method as claimed in claim 17 or claim
18, characterized in that anhydrous ethanol is
converted to ethylene.

21.    A method as claimed in any one of claims
17 to 19 wherein the ethanol is in vapour form
and is optionally in the presence of a further gas.

22.    A method as claimed in claim 17, characterized
in that aqueous ethanol comprising 4 to 96% v/v
ethanol contacts the catalyst at a temperature from
$250^O$ to $500^O$C at  pressure of 1 to 10 atmospheres
in an amount of 0.2 to 50 parts by weight of aqueous
ethanol per part by weight of catalyst per hour.

23.    A method as claimed in claim 17, characterized
in that aqueous ethanol comprising 4 to 96% v/v ethanol
contacts the catalyst at a temperature from $150^O$ to
$300^O$C at a pressure of 1 to 10 atmospheres in an
amount of 0.2 to 50 parts by weight of aqueous ethanol
per part by weight of catalyst per hour.

24.    A method as claimed in claim 17, characterized
in that anhydrous ethanol contacts the catalyst at
a temperature of $150^O$ to $300^O$C at a pressure of 1 to 10
atmospheres in an amount of 0.2 to 50 parts by weight
of aqueous  ethanol per part by weight of catalyst per
hour.

## DOCUMENTS CONSIDERED TO BE RELEVANT

| Category | Citation of document with indication, where appropriate, of relevant passages | Relevant to claim |
|---|---|---|
| | US − A − 4 044 065 (S.A. BUTTER) <br><br> * Claims 1,3,5,11,12 and 13; column 4, line 62 to column 5, line 23; example 1 * <br><br> --- | 1−4,6−9,14, 17,18 |
| | US − A − 4 011 278 (C.J. PLANK) <br><br> * Claims, examples * <br><br> --- | 1−4,6, 7,17 |
| | US − A − 4 025 571 (R.M. LAGO) <br><br> * Claims 1 and 5 to 9 * <br><br> ---- | 1−4,6, 7,17, 19 |

**CLASSIFICATION OF THE APPLICATION (Int. Cl.³)**

B 01 J 29/28
C 07 C 11/04
1/24
1/20
2/00

**TECHNICAL FIELDS SEARCHED (Int. Cl.³)**

B 01 J 29/06
29/28
C 07 C 11/04
1/24
1/20
2/00
2/76

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 22-10-1980 | THION |

EPO Form 1503.1   06.78